# EUROPEAN PATENT SPECIFICATION

(11) **EP 0 773 964 B1**
(45) Date of publication and mention of the grant of the patent: **14.02.2001**
(21) Application number: 95928287.2
(22) Date of filing: 02.08.1995
(51) Int. Cl.: C08F 210/16, C08F 36/08, C08F 36/06, C08F 2/34, C08F 4/70, C08F 4/64

(54) **GAS PHASE PRODUCTION OF POLYDIENES**
VERFAHREN ZUR HERSTELLUNG VON POLYDIENEN DURCH GASPHASENPOLYMERISATION
PRODUCTION EN PHASE GAZEUSE DE POLYDIENES

(30) Priority: 02.08.1994 US 284797
(43) Date of publication of application: 21.05.1997
(73) Proprietor: UNION CARBIDE CHEMICALS & PLASTICS TECHNOLOGY CORPORATION, Danbury, Connecticut 06817-0001 (US)
(72) Inventor: CANN, Kevin, Joseph, Rocky Hill, NJ 08522 (US); APECETCHE, Maria, Angelica, Piscataway, NJ 08854 (US); MOORHOUSE, John, Henry, Kendall Park, NJ 08824 (US); MURUGANANDAM, Natarajan, Somerville, NJ 08876 (US); SMITH, Gregory, George, Belle Mead, NJ 08502 (US); WILLIAMS, Gary, Harry, Flemington, NJ 08822 (US)
(74) Representative: Allard, Susan Joyce
(86) International application number: PCT/US95/09826
(87) International publication number: WO 96/04322

(56) References cited:
- EP-A- 0 456 437
- EP-A- 0 466 288
- EP-A- 0 535 582
- EP-A- 0 593 083
- US-A- 4 148 985
- US-A- 4 803 251
- US-A- 4 994 534

## Description

This application is a continuation-in-part-application of prior U.S. application, Serial No. 284,797, filed August 2, 1994.

### Field of the Invention

This invention relates to a process for producing polydienes in a gas phase reactor. More particularly, the invention relates to a process for making polybutadienes and polyisoprenes in a gas phase fluidized bed reactor.

### Background of the Invention

Polydienes such as polybutadiene and polyisoprene have been manufactured for many years by solution polymerization and more recently by mass or bulk polymerization processes. Various catalytic solution and bulk or mass processes for the polymerization of butadiene, particularly 1,3-butadiene, are known in the art to be suitable for producing polybutadiene with a high content of 1,4-cis units, which is particularly suited for the manufacture of tires, belting, and other molded or extruded rubber or elastomer articles.

In solution polymerization butadiene is polymerized in an inert solvent or diluent which does not enter into the structure of or adversely affect, the resulting polymer. Such solvents are usually aliphatic, aromatic and cycloaliphatic hydrocarbons such as pentane, hexane, heptane, benzene, toluene, cyclohexane and the like. The solvent/monomer volume ratio may be varied over a wide range. In bulk polymerizations, the reaction medium is substantially solventless, and the monomer is employed as a diluent.

The discovery of gas-phase fluidized bed and stirred reactor processes for the production of polymers, especially polyolefin polymers, made it possible to produce a wide variety of new polymers with highly desirable and improved properties. These gas-phase processes, especially the gas fluidized bed process for producing such polymers, provided a means for producing polymers with a drastic reduction in capital investment expense and dramatic savings in energy usage as compared to other then conventional polymerization processes.

In a conventional gas fluidized bed process a gaseous stream containing one or more monomers is passed into a fluidized bed reactor containing a bed of growing polymer particles in a polymerization zone, while continuously or intermittently introducing a polymerization catalyst into the polymerization zone. The desired polymer product is withdrawn from the polymerization zone, degassed, stabilized and packaged for shipment, all by well known techniques. Because the polymerization reaction is exothermic, substantial heat is generated in the polymerization zone which must be removed to prevent the polymer particles from overheating and fusing together. This is accomplished by continuously removing unreacted hot gases from the polymerization zone and replacing them with cooler gases. The hot gases removed from the polymerization zone are compressed, cooled in a heat exchanger, supplemented by additional amounts of monomer to replace monomer polymerized and removed from the reaction zone and then recycled into the bottom of the reactor. Cooling of the recycled gases is accomplished in one or more heat exchanger stages. The sequence of compression and cooling is a matter of design choice but it is usually preferable to provide for compression of the hot gases prior to cooling. The rate of gas flow into and through the reactor is maintained at a level such that the bed of polymer particles is maintained in a fluidized condition. The production of polymer in a stirred bed reactor is very similar, differing primarily in the use of mechanical stirring means to assist in maintaining the polymer bed in a fluidized or other well mixed state condition.

Conventional gas phase fluidized bed resin production is very well known in the art as shown, for example, by the disclosure appearing in United States Patent Nos. 4,379,758; 4,383,095 and 4,876,320.

The production of polymeric substances in gas phase stirred reactors is also well known in the art as exemplified by the process and equipment descriptions appearing in United States Patent No. 3,256,263.

More recently, in U.S. Patent Nos. 4,994,534 and 5,304,588, it has been taught that sticky polymers, including polybutadiene rubbers, can be produced in a fluidized bed reactor in the presence of a catalyst in a polymerization reaction above the softening temperatures of the sticky polymers in the presence of an inert particulate material. Preferably, the catalyst is a transition metal catalyst. The catalysts employed in the examples are titanium- and vanadium-based catalyst systems which also included a co-catalyst and optionally a promoter. The sticky polymers produced in the gas phase process are granular having a mixture of rubber and inert material with a core containing a majority of rubber while the shell contains a majority of inert material. Further, U.S. Patent No. 5,317,036 discloses gas phase polymerization processes which utilize unsupported, soluble catalysts such as, for example, transition metal coordination catalysts. The catalysts are introduced into the reactor, such as a fluidized bed, as a solution. The polyolefins produced by the process may contain dienes. EP 0 647 657 A1 discloses supported rare earth catalysts for gas phase polymerization of conjugated dienes.

For many years it was erroneously believed that to allow liquid of any kind to enter into the polymerization region of a gas phase reactor would inevitably lead to agglomeration of resin particles, formation of large polymer chunks and ultimately complete reactor shut-down. This concern caused gas phase polymer producers to carefully avoid cooling the recycle gas stream entering the reactor to a temperature below the condensation temperature of any of the monomers employed in the polymerization reaction.

Comonomers such as hexene-1, 4-methyl pentene and octene-1, are particularly valuable for producing ethylene copolymers. These higher alpha olefins have relatively high condensation temperatures. Due to the apprehension that liquid monomers in the polymerization zone would lead to agglomeration, chunking and ultimately shut down the reactor, production rates, which depend upon the rate at which heat is removed from the polymerization zone, were severely constrained by the perceived need to maintain the temperature of the cycle gas stream entering the reactor at temperature safely above the condensation temperature of the highest boiling monomer present in the cycle gas stream.

Even in the case of polymerization reactions conducted in stirred reactors, care was exercised to maintain the resin bed temperature above the condensation temperature of the recycle gas stream components.

To maximize heat removal it was not unusual to spray or inject liquid into or onto the polymer bed where it would immediately flash into a gaseous state by exposure to the hotter recycle gas stream. A limited amount of additional cooling was achieved by this technique by the Joules-Thompson effect but without ever cooling the recycle gas stream to a level where condensation might occur. This approach typically involved the laborious and energy wasting approach of separately cooling a portion of the cycle gas stream to obtain liquid monomer for storage and subsequent separate introduction into or onto the polymerization bed. Examples of this procedure are found in United States Patent Nos. 3,254,070; 3,300,457; 3,652,527 and 4,012,573.

It was discovered later, contrary to the long held belief that the presence of liquid in the cycle gas stream would lead to agglomeration and reactor shut-down, that it is indeed possible to cool the entire cycle gas stream to a temperature where condensation of significant amounts of monomer would occur without the expected dire results when these liquids were introduced into the reactor in temperature equilibrium with the recycle gas stream. Cooling the entire cycle gas stream produces a two-phase gas-liquid mixture in temperature equilibrium with each other so that the liquid contained in the gas stream does not immediately flash into vapor. Instead a substantially greater amount of cooling takes place because the total mass of both gas and liquid enters the polymerization zone at a substantially lower temperature than previously thought possible. This process led to substantial improvements in the yield of polymers produced in the gas phase, especially where comonomers which condense at relatively low temperatures are used. This procedure, commonly referred to as "condensing mode" operation, is described in detail in United States Patent Nos. 4,543,399 and 4,588,790. In condensing mode operation the two-phase gas-liquid mixture entering the polymerization zone is heated quite rapidly and is completely vaporized within very short distance after entry into the polymerization zone. Even in the largest commercial reactors, all liquid has been vaporized and the temperature of the then totally gaseous cycle gas stream raised substantially by the exothermic nature of the polymerization reaction soon after entry into the polymerization zone. The ability to operate a gas phase reactor in condensing mode was believed possible due to the rapid heating of the two-phase gas liquid stream entering the reactor coupled with efficient constant back mixing of the fluidized bed leaving no liquid present in the polymer bed more than a short distance above the entry level of the two-phase gas-liquid recycle stream.

We have now found that liquid monomer may be present throughout the entire polymer bed provided that the liquid monomer present in the bed is adsorbed on or absorbed in solid particulate matter present in the bed, such as the polymer being produced or fluidization aids present in the bed, so long as there is no substantial amount of free liquid monomer. This discovery makes it possible to produce polymers in a gas phase reactor with the use of monomers having condensation temperatures much higher than the temperatures at which conventional polyolefins are produced in gas phase reactors. Another way of viewing this discovery is that it is now possible to produce polymers using readily condensible monomers (e.g., 1,3-butadiene, having a normal boiling point of -4.5°C) in a gas phase reactor under conditions at which the monomer would be expected to be present as a liquid. Furthermore, it had been previously believed that gas phase processes for producing polymers with some or all of the monomers having low to moderate condensation temperatures were impractical because the amount of polymer produced per catalyst particle was too low at all monomer concentrations that had condensation temperatures below the temperature in the polymerization zone. The discovery of this invention now makes it economically practical to produce polymer with monomers at concentrations where they have condensation temperatures higher than the temperature in the polymerization zone, such that liquid monomer is present throughout the entire polymer bed provided that the liquid monomer present in the bed is adsorbed on or absorbed in solid particulate matter, the polymer bed, and/or the forming polymer product present in the polymerization zone of the reactor. This invention makes possible the gas phase production of classes of polymers which previously were thought not capable of production in a continuous gas phase process.

Another benefit of the invention is that operation with monomer present as liquid dissolved in the polymer gives a greater concentration of monomer at the active catalyst site than operation with monomer not dissolved, i.e., present only in the gas phase. This should maximize the productivity of the catalyst for making polymer. Still another benefit of the invention is that heat transfer within the polymer particles should be improved due to removal of heat by monomer evaporation. This should lead to more uniform polymer particle temperatures, more uniform polymer properties, less polymer fouling, and possibly improved polymer morphology than operation with monomer not dissolved, i.e., present only in the gas phase.

### Summary of the Invention

The present invention provides a process for producing polybutadiene or polyisoprene in a stirred bed or gas fluidized polymerization vessel having a polymerization zone under polymerization reaction conditions, which process comprises:
(i) introducing butadiene or isoprene monomer into said polymerization zone containing a bed of growing polymer particles in the presence of an inert particulate material and optionally at least one inert gas;
(ii) continuously or intermittently introducing a polymerization catalyst containing a metal component of nickel, cobalt, titanium, or mixtures thereof, a co-catalyst, and optionally a promoter into said polymerization zone;
(iii) continuously or intermittently withdrawing polybutadiene or polyisoprene product from said polymerization zone; and
(iv) withdrawing unreacted butadiene or isoprene from said polymerization zone, compressing and cooling said butadiene or isoprene and said inert gas when present, while maintaining the temperature within said polymerization zone below the dew point of the monomer present in said polymerization zone.

Granular particles and articles produced using such particles are also provided.

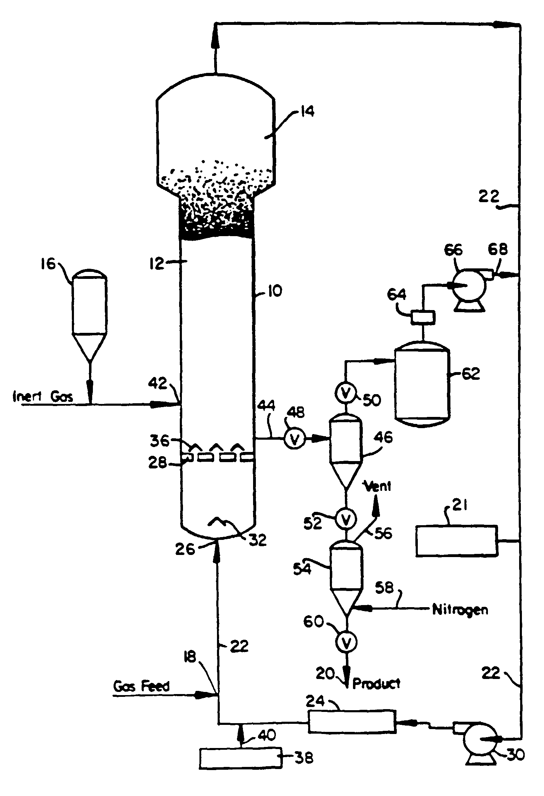

### Brief Description of the Drawing

A fluidized bed reaction system which is particularly suited to the production of polybutadiene and polyisoprene is illustrated in the drawing.

### Detailed Description Of The Invention

While not limited to any particular type or kind of polymerization reaction, this invention is particularly well suited to olefin polymerization reactions involving homopolymerization and copolymerization of relatively high boiling or readily condensable monomers such as polybutadiene and polyisoprene.

Examples of higher boiling or readily condensable monomers capable of undergoing olefinic polymerization reactions are the following:
A. higher molecular weight alpha olefins such as decene-1, dodecene-1, isobutylene, styrene and the like.
B. dienes such as hexadiene, vinyl cyclohexene, dicyclopentadiene, butadiene, isoprene, ethylidene norbornene and the like.
C. polar vinyl monomers such as acrylonitrile, maleic acid esters, vinyl acetate, acrylate esters, methacrylate esters, vinyl trialkyl silanes and the like.

These higher boiling or readily condensable monomers can be homopolymerized in accordance with this invention with the use of an inert gas as a gaseous component of the two phase gas-liquid mixture cycled through the reactor. Suitable inert materials for this purpose include nitrogen, argon, and saturated hydrocarbons which remain gaseous at a temperature below the temperature selected to be maintained in the polymerization zone.

The higher boiling or readily condensable monomers can also be copolymerized with one or more lower boiling monomers such as ethylene, propylene and butene, as well as with other higher boiling monomers such as those mentioned above, the only requirement being that there be enough gas present in the cycle gas stream to permit practical, steady state, continuous operation. Accordingly, styrene and butadiene can be copolymerized to produce styrene-butadiene rubber (SBR) in accordance with the process of the present invention.

In accordance with our invention the higher boiling or readily condensable monomers can be directly introduced into the polymerization zone or carried into the polymerization zone as with the recycle gas stream or a combination of both. In a preferred embodiment the temperature within said polymerization zone is maintained below the condensation temperature of the monomer (e.g., 1,3-butadiene or isoprene) present in said polymerization zone. In another embodiment the conditions (e.g., temperature, pressure, monomer(s) concentration) within said polymerization zone are such that essentially no liquid is present in said polymerization zone that is not adsorbed on or absorbed in solid particulate matter. Alternatively, the conditions within said polymerization zone are maintained such that a portion of the monomer in the polymerization zone is a liquid that is not absorbed in solid particulate matter.

The practice of this invention is not limited to any particular class or kind of catalyst. Any catalyst useful in the conduct of gas phase polymerization reactions is suitable for use in the practice of this invention. Catalysts that have been previously employed in slurry, solution, or bulk polymerizations of higher boiling or readily condensable monomers (e.g., butadiene and isoprene) can also be utilized in this invention.

The conventional Ziegler-Natta catalysts, by which is meant those formed by reacting a metal alkyl or hydride with a transition metal compound, are preferred in the practice of this invention. Those formed by reacting an aluminum alkyl with salts of metals of groups I to III of periodic table are particularly useful.

Illustrative of the catalysts useful in the practice of this invention are the following:
A. Titanium based catalysts such as those described in U.S. Patent Nos. 4,376,062; 4,379,758.
B. Chromium based catalysts such as those described in U.S. Patent Nos. 3,709,853; 3,709,954; and 4,077,904.
C. Vanadium based catalysts such as vanadium oxychloride and vanadium acetylacetonate.
D. Metallocene catalysts such as those described in U.S. Patent Nos. 4,530,914; 4,665,047; 4,752,597; 5,218,071; 5,272,236; and 5,278,272.
E. Cationic forms of metal halides, such as aluminum trihalides.
F. Cobalt catalysts and mixture thereof such as those described in U.S. Patent Nos. 4,472,559 and 4,182,814.
G. Nickel catalysts and mixtures thereof such as those described in U.S. Patent Nos. 4,155,880 and 4,102,817.

The transition metal catalysts employed in the process of this invention can have a metal component, a co-catalyst, and optionally a promoter. The metal component can be a transition metal compound or a mixture of two or more transition metal compounds. In general, the transition metal component of the catalyst can be soluble or insoluble, supported or unsupported, or spray dried in either the presence or absence of a filler. Alternatively, the polymerization catalyst can be introduced to the polymerization zone in the form of a prepolymer using techniques known to those skilled in the art.

When the metal component is supported, typical supports can include, for example, silica, carbon black, porous crosslinked polystyrene, porous crosslinked polypropylene, alumina, thoria, zirconia, or magnesium halide (e.g., magnesium chloride) support materials or a mixture thereof. Silica, carbon black, and alumina are preferred support materials. Silica and carbon black are the most preferred support materials. A typical silica or alumina support is a solid, particulate, porous material essentially inert to the polymerization. It is used as a dry powder having an average particle size of about 10 to about 250 µm and preferably about 30 to about 100 µm; a surface area of at least 200 square meters per gram and preferably at least about 250 square meters per gram; and a pore size of at least about 100 Å and preferably at least about 200 Å. Generally, the amount of support used is that which will provide about 0.1 to about 1.0 millimole of transition metal per gram of support. In a preferred embodiment, two types of carbon black are used as support. DARCO G-60 (pH of water extract = 5) is used as dry powder having a surface area of 505 square meters per gram, average particle size of 100 µm, and porosity of 1.0 to 1.5 cubic centimeter per gram. NORIT A (pH of water extract = 9 - 11) used as a dry powder has a surface area of 720 square meters per gram, average particle size of 45 to 80 µm.

The metal component can be impregnated on a support by well known means such as by dissolving the metal compound in a solvent or diluent such as a hydrocarbon or tetrahydrofuran in the presence of the support material and then removing the solvent or diluent by evaporation such as under reduced pressure. Alternatively, the transition metal component can be dissolved in a solvent or diluent such as a hydrocarbon or tetrahydrofuran and spray dried to generate a well-shaped catalyst precursor having little or no silica or other inorganic solids content, if desired.

The preferred transition metal compounds for making polybutadiene and polyisoprene are compounds containing nickel, titanium, and cobalt, with cobalt and nickel compounds being the most preferred. Nickel compounds of the metal component of the catalyst are organonickel compounds of nickel with mono- or bi-dentate organic ligands containing up to 20 carbon atoms. "Ligand" is defined as an ion or molecule bound to and considered bonded to a metal atom or ion. Mono-dentate means having one position through which covalent or coordinate bonds with the metal may be formed; bi-dentate means having two positions through which covalent or coordinate bonds with the metal may be formed. The organonickel compounds are generally soluble in inert solvents. Thus, any salt or an organic acid containing from about 1 to 20 carbon atoms may be employed. Representative of organonickel compounds are nickel benzoate, nickel acetate, nickel naphthenate, nickel octanoate, nickel neodecanoate, nickel 2-ethylhexanoate, bis(π-allyl nickel), bis(π-cycloocta-1,5-diene), bis(π-allyl nickel trifluoroacetate), bis(α-furyl dioxime) nickel, nickel palmitate, nickel stearate, nickel acetylacetonate, nickel salicaldehyde, bis(salicyladehyde) ethylene diimine nickel, bis(cyclopentadiene) nickel, cyclopentadienylnickel nitrosyl and nickel tetracarbonyl. The preferred component containing nickel is a nickel salt of a carboxylic acid or an organic complex compound of nickel.

Co-catalysts that can be employed with the component containing nickel include triethylaluminum (TEAL), triisobutylaluminum (TIBA), diethyl aluminum chloride (DEAC), partially hydrolyzed diethyl aluminum chloride (DEACO), methylaluminoxane (MAO), or modified methylaluminoxane (MMAO) or a mixture thereof.

When MAO or MMAO is employed as the co-catalyst, it may be one of the following: (a) branched or cyclic oligomeric poly(hydrocarbylaluminum oxide)s which contain repeating units of the general formula -(Al(R"')O)-, where R''' is hydrogen, an alkyl radical containing from 1 to about 12 carbon atoms, or an aryl radical such as a substituted or unsubstituted phenyl or naphthyl group; (b) ionic salts of the general formula [A⁺][BR^{*}₄⁻], where A⁺ is a cationic Lewis or Bronsted acid capable of abstracting an alkyl, halogen, or hydrogen from the transition metal component of the catalyst, B is boron, and R* is a substituted aromatic hydrocarbon, preferably a perfluorophenyl radical; and (c) boron alkyls of the general formula BR*₃, where R* is as defined above.

Aluminoxanes are well known in the art and comprise oligomeric linear alkyl aluminoxanes represented by the formula: 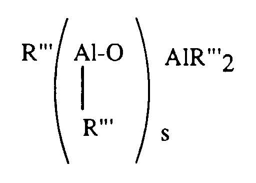 and oligomeric cyclic alkyl aluminoxanes of the formula: 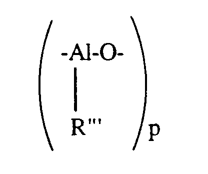 wherein s is 1 to 40, preferably 10 to 20; p is 3 to 40, preferably 3 to 20; and R"' is an alkyl group containing 1 to 12 carbon atoms, preferably methyl or an aryl radical such as a substituted or unsubstituted phenyl or naphthyl radical. Modified methylaluminoxane is formed by substituting 20-80 wt % of the methyl groups with a C₂ to C₁₂ group, preferably with isobutyl groups, using techniques known to those skilled in the art.

Promoters that can be used with the component containing nickel include hydrogen fluoride (HF), borontrifluoride (BF₃), an etherate of HF and/or BF₃ or a mixture thereof.

The titanium compound (titanates) can be TiCl₄, TiBr₄, TiI₄ or Ti(OR)₄ wherein R is an alkyl radical.

Co-catalysts that can be employed with the component containing titanium include TEAL, TIBA, dialkylaluminum iodide, MAO or a mixture thereof.

Promoters that can be used with the component containing titanium include iodine and organic etherates. For isoprene, the combination TiCl₄, TIBA, and DPE (diphenyl ether) is employed.

The cobalt compound can be any organic compound such as the cobalt salts of organic acids, cobalt complexes and the like. Preferably, the cobalt compound is selected from the group consisting of cobalt β-ketone complexes, for example, cobalt (II) acetylacetonate and cobalt (III) acetylacetonate; cobalt β-ketoacid ester complexes, for example, cobalt acetylacetonate ethylester complexes; cobalt salts of organic carboxylic acids having 6 or more carbon atoms, for example, cobalt octoate, cobalt naphthenate, and cobalt benzoate; and cobalt halide complexes, for example, cobalt chloride-pyridine complexes; cobalt chloride-ethyl alcohol complexes and cobalt complexes coordinated with butadiene, for example, (1,3-butadiene) [1-(2-methyl-3-butenyl)- π-allyl]-cobalt which may be prepared, for example, by mixing a cobalt compound with an organic aluminum compound, organic lithium compound or alkyl magnesium compound and 1,3-butadiene. Other typical cobalt compounds are cobalt sorbate, cobalt adipate, cobalt 2-ethylhexoate, cobalt stearate, and the like compounds wherein the organic portion of the molecule contains about 5 to 20, preferably 8 to 18 carbon atoms and one or two carboxylic functions, as well as acetylacetonate.

Co-catalysts that can be employed with the component containing cobalt include ethylaluminum sesquichloride (EASC), ethylaluminum dichloride (EADC), DEACO, diisobutyl aluminum chloride, partially hydrolized diisobutylaluminum chloride, diethylaluminum chloride, MAO and mixtures thereof.

Water in small amounts can be used as a promoter with the metal component containing cobalt, if desired.

Fluidization aids that can be employed in the invention are inert particulate materials which are chemically inert to the reaction. Examples of such fluidization aids include carbon black, silica, clays, talc or a mixture thereof. Organic polymeric materials can also be employed as a fluidization aid. Carbon blacks and silicas are the preferred fluidization aids with carbon black being the most preferred. The carbon black materials employed have a primary particle size of about 10 to 100 nm and an average size of aggregate (primary structure) of about 0.1 to about 10 µm. The specific surface area of the carbon black is about 30 to 1,500 m2/gm and the carbon black displays a dibutylphthalate (DBP) absorption of about 80 to about 350 cc/100 grams.

Silicas which can be employed are amorphous and have a primary particle size of about 5 to 50 nm and an average size of aggregate of about 0.1 to 10 µm. The average size of agglomerates of silica is about 2 to about 120 µm. The silicas employed have a specific surface area of about 50 to 500 m2/gm and a dibutylphthalate (DBP) absorption of about 100 to 400 cc/100 grams.

Clays which can be employed according to the invention have an average particle size of about 0.01 to about 10 µm and a specific surface area of about 3 to 30 m2/gm. They exhibit oil absorption of about 20 to about 100 gms per 100 gms.

Organic polymeric substances which can be used include polymers and copolymers of ethylene, propylene, butene, and other alpha olefins and polystyrene, in granular or powder form. These organic polymeric materials have an average particle size ranging from about 0.01 to 100 µm, preferably 0.01 to 10 µm.

In general, the amount of fluidization aid utilized generally depends on the type of material utilized and the type of polybutadiene or polyisoprene produced. When utilizing a fluidization aid such as carbon black and silica, they can be employed in amounts of about 0.3% to about 80% by weight, preferably about 5% to about 60%, and most preferably about 10 to about 45% based on the weight of the final product (polybutadiene or polyisoprene) produced. Typically, when clays or talcs are employed as the fluidization aid, the amount can range from about 0.3% to about 80% based on the weight of the final product, preferably about 12% to 75% by weight. Organic polymeric materials are used in amounts of about 0.1% to about 50% by weight, preferably about 0.1% to about 10% based on the weight of the final polymer product produced.

The fluidization aid can be introduced into the reactor at or near the top of the reactor, at the bottom of the reactor, or to the recycle line directed into the bottom of the reactor. Preferably, the fluidization aid is introduced at or near the top of the reactor or above the fluidized bed. It is preferred to treat the fluidization aid prior to entry into the reactor to remove traces of moisture and oxygen. This can be accomplished by purging the material with nitrogen gas and heating by conventional procedures. The fluidization aid can be added separately or combined with one or more monomers, or with a soluble unsupported catalyst. Preferably, the fluidization aid is added separately.

A fluidized bed reaction system which is particularly suited to production of polymeric materials in accordance with the present invention is illustrated in the drawing. With reference thereto, the reactor 10 consists of a reaction zone 12 and a velocity reduction zone 14.

In general, the height to diameter ratio of the reaction zone can vary in the range of about 2.7:1 to about 4.6:1. The range, of course, can vary to larger or smaller ratios and depends upon the desired production capacity. The cross-sectional area of the velocity reduction zone 14 is typically within the range of about 2.6 to about 2.8 multiplied by the cross-sectional area of the reaction zone 12.

The reaction zone 12 includes a bed of growing polymer particles, formed polymer particles and a minor amount of catalyst particles fluidized by the continuous flow of polymerizable and modifying gaseous components in the form of make-up feed and recycle fluid through the reaction zone. To maintain a viable fluidized bed, the superficial gas velocity through the bed must exceed the minimum flow required for fluidization, and preferably is at least 0.1 ft./sec (1 ft/sec ≙ 0,305 m/sec) above minimum flow. Ordinarily, the superficial gas velocity does not exceed 5.0 ft./sec and usually no more than 2.5 ft./sec is sufficient.

It is essential that the bed always contain particles to prevent the formation of localized "hot spots" and to entrap and distribute catalyst throughout the reaction zone. On start up, the reactor is usually charged with a bed of particulate polymer particles. Such particles may be identical in nature to the polymer to be formed or they may be different. When different, they are withdrawn with the desired formed polymer particles as the first product. Eventually, a fluidized bed of desired polymer particles supplants the start-up bed.

A partially or totally activated precursor composition and/or catalyst used in the fluidized bed is preferably stored for service in a reservoir 16 under a blanket of a gas which is inert to the stored material, such as nitrogen or argon.

Fluidization is achieved by a high rate of fluid recycle to and through the bed, typically on the order of about 50 to 150 times the rate of feed of make-up fluid. The fluidized bed has the general appearance of a dense mass of individually moving particles as created by the percolation of gas through the bed. The pressure drop through the bed is equal to or slightly greater than the weight of the bed divided by the cross-sectional area. It is thus dependent on the geometry of the reactor.

Make-up fluid can be fed to the bed at point 18. The composition of the make-up stream is determined by a gas analyzer 21. The gas analyzer determines the composition of the recycle stream and the composition of the make-up stream is adjusted accordingly to maintain an essentially steady state gaseous composition within the reaction zone.

The gas analyzer is a conventional gas analyzer which operates in a conventional manner to determine the recycle stream composition to facilitate maintaining the ratios of feed stream components. Such equipment is commercially available from a wide variety of sources. The gas analyzer 21 is typically positioned to receive gas from a sampling point located between the velocity reduction zone 14 and heat exchanger 24.

The higher boiling monomers can be introduced into the polymerization zone in various ways including direct injection through a nozzle (not shown in the drawing) into the bed or by spraying onto the top of the bed through a nozzle (not shown) positioned above the bed, which may aid in eliminating some carryover of fines by the cycle gas stream. If the rate of monomer feed is relatively small, heavier monomers can be introduced into the polymerization zone simply by suspension in the cycle gas stream entering the bottom of the reactor.

To ensure complete fluidization, the recycle stream and, where desired, part of the make-up stream are returned through recycle line 22 to the reactor at point 26 below the bed. There is preferably a gas distributor plate 28 above the point of return to aid in fluidizing the bed. In passing through the bed, the recycle stream absorbs the heat of reaction generated by the polymerization reaction.

The portion of the fluidizing stream which has not reacted in the bed is removed from the polymerization zone, preferably by passing it into velocity reduction zone 14 above the bed where entrained particles can drop back into the bed.

The recycle stream is compressed in a compressor 30 and then passed through a heat exchange zone where heat is removed before it is returned to the bed. The heat exchange zone is typically a heat exchanger 24 which can be of the horizontal or vertical type. If desired, several heat exchangers can be employed to lower the temperature of the cycle gas stream in stages. It is also possible to locate the compressor downstream from the heat exchanger or at an intermediate point between several heat exchangers. After cooling, the recycle stream is returned to the reactor at its base 26 and to the fluidized bed through gas distributor plate 28. A gas deflector 32 can be installed at the inlet to the reactor to prevent contained polymer particles from settling out and agglomerating into a solid mass, and to prevent liquid accumulation at the bottom of the reactor, as well to facilitate easy transitions between processes which contain liquid in the cycle gas stream and those which do not and vice versa. Illustrative of gas deflectors suitable for this purpose is the apparatus described in U.S. Patent No. 4,933,149.

The selected temperature of the bed is maintained at an essentially constant temperature under steady state conditions by constantly removing the heat of reaction. No noticeable temperature gradient appears to exist within the upper portion of the bed. A temperature gradient can exist in the bottom of the bed in a layer of about 15.24 to 30.48 cm (6 to 12 inches), between the temperature of the inlet fluid and the temperature of the remainder of the bed.

Good gas distribution plays an important role in the operation of the reactor. The fluidized bed contains growing and formed particulate polymer particles, as well as catalyst particles. As the polymer particles are hot and possibly active, they must be prevented from settling, for if a quiescent mass is allowed to exist, any active catalyst contained therein may continue to react and cause fusion. Diffusing recycle fluid through the bed at a rate sufficient to maintain fluidization throughout the bed is, therefore, important.

Gas distribution plate 28 is a preferred means for achieving good gas distribution and may be a screen, slotted plate, perforated plate, a plate of the bubble-cap type and the like. The elements of the plate may all be stationary, or the plate may be of the mobile type disclosed in U.S. 3,298,792. Whatever its design, it must diffuse the recycle fluid through the particles at the base of the bed to keep the bed in a fluidized condition, and also serve to support a quiescent bed of resin particles when the reactor is not in operation.

The preferred type of gas distributor plate 28 is metal and has holes distributed across its surface. The holes are normally of a diameter of about 1.27 cm (1/2 inch). The holes extend through the plate. Over each hole there is positioned a triangular angle iron identified as 36 which is mounted on plate 28. The angle irons serve to distribute the flow of fluid along the surface of the plate so as to avoid stagnant zones of solids. In addition they prevent the polymer from flowing through the holes when the bed is settled.

Any fluid inert to the catalyst and reactants can also be present in the recycle stream. An activator compound, if utilized, is preferably added to the reaction system downstream from heat exchanger 24, in which case the activator may be fed into the recycle system from dispenser 38 through line 40.

In the practice of this invention operating temperatures can extend over a range of from about -100°C to about 150°C with temperatures ranging from about 20°C to about 120°C being preferred.

The fluid-bed reactor can be operated at pressures up to about 6.89 x 10³ kPa (1000 psi) and preferably at a pressure of from about 689 kPa (100 psi) to about 4134 kPa (600 psi). Operation at higher pressures favors heat transfer as an increase in pressure increases the unit volume heat capacity of the gas.

The partially or totally activated precursor composition and/or catalyst (hereinafter collectively referred to as catalyst) is injected into the bed at a rate equal to its consumption at a point 42 which is above distributor plate 28. Preferably, the catalyst is injected at a point in the bed where good mixing with polymer particles occurs. Injecting the catalyst at a point above the distribution plate provides for satisfactory operation of a fluidized bed polymerization reactor. Injection of the catalyst into the area below the distributor plate could cause polymerization to begin there and eventually cause plugging of the distributor plate. Injection directly into the fluidized bed aids in distributing the catalyst uniformly throughout the bed and tends to avoid the formation of localized spots of high catalyst concentration which can cause "hot spots" to form. Injection of the catalyst into the reactor above the bed can result in excessive catalyst carryover into the recycle line where polymerization can occur leading to plugging of the line and heat exchanger.

For a supported catalyst, it can be injected into the reactor by various techniques. It is preferred, however, to continuously feed the catalyst into the reactor utilizing a catalyst feeder as disclosed, e.g., in U.S. Patent No. 3,779,712. For a catalyst in solution, liquid, or slurry form, it is preferably fed in accordance with the disclosure in U.S. Patent No. 5,317,036 to Brady *et al.* and U.S. Serial No. 414,522 entitled "Process for Controlling Particle Growth During Production of Sticky Polymers, filed March 31,1995. The catalyst is preferably fed into the reactor at a point 20 to 40 percent of the reactor diameter away from the reactor wall and at a height of about 5 to about 30 percent of the height of the bed.

A gas which is inert to the catalyst, such as nitrogen or argon, is preferably used to carry the catalyst into the bed.

The rate of polymer production in the bed depends on the rate of catalyst injection and the concentration of monomer(s) in the reactor stream. The production rate is conveniently controlled by simply adjusting the rate of catalyst injection.

Since any change in the rate of catalyst injection will change the reaction rate and thus the rate at which heat is generated in the bed, the temperature of the recycle stream entering the reactor is adjusted upwards and downwards to accommodate any change in the rate of heat generation. This ensures the maintenance of an essentially constant temperature in the bed. Complete instrumentation of both the fluidized bed and the recycle stream cooling system is, of course, useful to detect any temperature change in the bed so as to enable either the operator or a conventional automatic control system to make a suitable adjustment in the temperature of the recycle stream.

Under a given set of operating conditions, the fluidized bed is maintained at essentially a constant height by withdrawing a portion of the bed as product at the rate of formation of the particulate polymer product. Since the rate of heat generation is directly related to the rate of product formation, a measurement of the temperature rise of the fluid across the reactor (the difference between inlet fluid temperature and exit fluid temperature) is indicative of the rate of particular polymer formation at a constant fluid velocity if no or negligible vaporizable liquid is present in the inlet fluid.

On discharge of particulate polymer product from reactor 10, it is desirable and preferable to separate fluid from the product and to return the fluid to the recycle line 22. There are numerous ways known to the art to accomplish this. One preferred system is shown in the drawings. Thus, fluid and product leave reactor 10 at point 44 and enter product discharge tank 46 through valve 48, which may be a ball valve which is designed to have minimum restriction to flow when opened. Positioned above and below product discharge tank 46 are conventional valves 50, 52 with the latter being adapted to provide passage of product into product surge tank 54. Product surge tank 54 has venting means illustrated by line 56 and gas entry means illustrated by line 58. Also positioned at the base of product surge tank 54, is a discharge valve 60 which when in the open position discharges product for conveying to storage. Valve 50 when in the open position releases fluid to surge tank 62. Fluid from surge tank 62 is directed through a filter absorber 64 and thence through a compressor 66 and into recycle line 22 through line 68.

In a typical mode of operation, valve 48 is open and valves 50, 52 are in a closed position. Product and fluid enter product discharge tank 46. Valve 48 closes and the product is allowed to settle in product discharge tank 46. Valve 50 is then opened permitting fluid to flow from product discharge tank 46 to surge tank 62 from which it is continually compressed back into recycle line 22. Valve 50 is then closed and valve 52 is opened and any product in product discharge tank 46 flows into product surge tank 54. Valve 52 is then closed. The product is purged with inert gas, preferably nitrogen, which enters product surge tank 54 through line 58 and is vented through line 56. Product is then discharged from product surge tank 54 through valve 60 and conveyed through line 20 to storage.

The particular timing sequence of the valves is accomplished by the use of conventional programmable controllers which are well known in the art. Moreover, the valves can be kept substantially free of agglomerated particles by directing a stream of gas periodically through the valves and back to the reactor.

Another preferred product discharge system which may be alternatively employed is that disclosed and claimed in the copending U.S. patent application of Robert G. Aronson filed July 28, 1981, Ser. No. 287,815 and entitled "Fluidized Bed Discharge System", now U.S. Patent No. 4,621,952. Such a system employs at least one (parallel) pair of tanks comprising a settling tank and a transfer tank arranged in series and having the separated gas phase returned from the top of the settling tank to a point in the reactor near the top of the fluidized bed. Such alternative preferred product discharge system obviates the need for a recompression line 64, 66, 68, as shown in the system of the drawing.

The fluidized-bed reactor is equipped with an adequate venting system (not shown) to allow venting the bed during start up and shut down. The reactor does not require the use of stirring and/or wall scraping. The recycle line 22 and the elements therein (compressor 30, heat exchanger 24) should be smooth surfaced and devoid of unnecessary obstructions so as not to impede the flow of recycle fluid or entrained particles.

Illustrative of the polymers which can be produced in accordance with the invention are the following:
Polyisoprene
Polybutadiene
SBR (polymer of butadiene copolymerized with styrene)

When it is desired to produce polymers or copolymers using one or more monomers which are all relatively high boiling or readily condensable and which form liquids under the temperature and pressure conditions which are preferred for gas phase fluidized bed production in accordance with the invention, it is preferable to employ an inert substance which will remain gaseous under the conditions selected for polymerization in the fluidized bed. Suitable for this purpose are inert gases such as nitrogen, argon, neon, krypton and the like. Also useful are saturated hydrocarbons such as ethane, propane, butane and the like as well as halogen substituted alkanes such as freon. Other materials which remain gaseous under the desired conditions, such as carbon dioxide, provided they are essentially inert and do not affect catalyst performance, can also be employed.

Nitrogen, because of its physical properties and relatively low cost is a preferred medium for the manufacture of polymers from higher boiling monomers such as styrene, vinyl acetic acid, acrylonitrile, methylacrylate, methylmethacrylate and the like. Alkanes such as ethane and propane which remain gaseous at relatively low temperatures are also preferred.

Conventional techniques for the prevention of fouling of the reactor and polymer agglomeration can be used in the practice of our invention. Illustrative of these techniques are the introduction of finely divided particulate matter to prevent agglomeration, as described in U.S. Patent Nos. 4,994,534 and 5,200,477; the addition of negative charge generating chemicals to balance positive voltages or the addition of positive charge generating chemicals to neutralize negative voltage potentials as described in U.S. Patent No. 4,803,251. Antistat substances may also be added, either continuously or intermittently to prevent or neutralize static charge generation.

The granular polybutadiene and/or polyisoprene elastomers of this invention can be compounded with other elastomers, e.g. natural rubber, styrene-butadiene rubber, (halo)butyl rubber, ethylene-propylene-diene rubber; reinforcing fillers, e.g. carbon black, silica; processing aids, antidegradants, and vulcanizing agents, using equipment and methods well known to those skilled in the art. In such compounds, the initially granular form of the polybutadiene or polyisoprene may permit more intimate mixing with the other elastomer(s), than would be achievable with conventional polybutadiene or polyisoprene in solid bale form. It is generally desirable that elastomer blends be intimately mixed in order to optimize the mechanical properties of the vulcanizate. Furthermore, if the inert particulate material of this invention, used as an aid to maintain granularity during and after the polymerization process, also happens to be a reinforcing filler for the compound (e.g., carbon black), then a further benefit may be realized in the form of a shorter mixing time required to disperse the filler in the compound. This is because the filler, which normally would have to first be deagglomerated in the mixing process before it could be dispersed, in this case enters the mixing process already substantially deagglomerated and dispersed.

Elastomeric compounds prepared from granular, relatively free flowing, as compared to a bale form, polybutadiene and/or polyisoprene, as described in the preceding paragraph, are particularly useful as components of pneumatic tires. For example, in the production of a radial automobile tire, specially formulated elastomeric compounds can be extruded through a die to produce strip stock for the tread, sidewall, and bead filler components of the tire, or to produce sheet stock for the air retention innerliner. Other specially formulated elastomeric compounds can be calendered onto textile or steel cord fabric to produce cord-reinforced sheet stock for the carcass and circumferential belt components of the tire. The "green" or unvulcanized tire is built by assembling the various components (except circumferential belt and tread) on the surface of a cylindrical drum, radially expanding and axially compressing the assembly to produce a toroidal shape, then placing the belt and tread components in position around the circumference of the toroid. Finally, the green tire is vulcanized by inflating with high pressure steam against the inner surface of a closed, heated aluminum mold. In the early stage of the vulcanization process, when the various elastomeric compounds are still soft and flowable, the pressure of the tire against the inner surface of the mold produces the final precise shape, tread pattern, sidewall lettering and decorative markings. Later in the vulcanization process, heat-activated crosslinking reactions take place within the various elastomeric compounds, so that when the mold is finally opened, each compound has undergone crosslinking to a degree that is essentially optimum for the intended service.

When used as a constituent of tire compounds, granular polybutadiene of this invention particularly imparts abrasion resistance, fatigue cracking resistance, low heat generation, and low rolling resistance. Granular polyisoprene of this invention particularly imparts building tack and green strength, which facilitate the building and handling of the green tire, and tear and cut resistance. The granular, free-flowing polybutadiene and polyisoprene produced in the gas phase process of the present invention can also be employed in other molded or extruded articles using techniques known to those skilled in the art. Granular, relatively free-flowing SBR produced in the gas phase process of the present invention is employed in molded or extruded articles using techniques known to those skilled in the art.

The following examples are provided to illustrate our invention.

### Example 1 (Comparative)

In an example of the process of the invention a fluidized bed reaction system as described above, was operated as described below to produce ethylene-propylene diene terpolymer. The polymer was produced under the following reaction conditions: 40°C reactor temperature and 1998 kPa (290 psia) reactor pressure. The partial pressures (dew points) of the monomers and comonomers inside the reactor were 620 kPa (90 psia) for ethylene and 1364 kPa (198 psia) for propylene. The partial pressure of hydrogen was 13.78 kPa (2.0 psia). The monomer ethylidenenorbornene (ENB) was injected into the polymerization zone of the reactor at the rate of 1.16 kg/h (0.53 lb/h). The volume of the reactor was 1.54 m³ (55 ft³); the resin's weight inside the reactor was 50.8 kg (112 lbs). The catalyst system employed in this Example was vanadium acetyl acetonate with diethylaluminum chloride as co-catalyst and ethyl trichloroacetate as the promoter. The production rate was 9.08 kg/h (20 lb/h). The product had a Mooney value of 55.

Seventy-five percent of the injected ENB was incorporated into the polymers by polymerization. The unreacted remainder of ENB, dissolved into polymers and was equal to 0.66 percent of the polymer's weight. With 50.8 kg (112 lbs) of resins inside the reactor, the total unreacted ENB was 1.63 kg (0.74 lbs). If the unreacted ENB were completely evaporated inside the reactor, its partial pressure would be 4.66 kPa (0.6764 psia).

At 40°C the saturation pressure is 15.073 x 10³ kPa (2187.7 psia) for ethylene, 2323 kPa (337.1 psia) for propylene and 1.80 kPa (0.262 psia) for ENB. Since the partial pressures of ethylene and propylene inside the reactor were much less than their saturation pressures, there was no condensed ethylene or propylene. The calculated partial pressure of unreacted ENB inside the reactor, however, is much higher than its saturation pressure. Therefore, the ENB must have remained in a liquid state and been absorbed by the polymers.

### Example 2 (comparative)

Ethylene-propylene diene terpolymer was made in a fluidized bed reaction system as described above under the following reaction conditions: 40°C reactor temperature and 2503.8 kPa (363.4 psia) reactor pressure. The partial pressures of the monomers and comonomers inside the reactor were 620 kPa (90 psia) for ethylene and 1364 kPa (198.2 psia) for propylene. The partial pressure of hydrogen was 15.16 kPa (2.2 psia), and the partial pressure of nitrogen was 72.6. The monomer ethylidenenorbornene (ENB) was injected into the polymerization zone of the reactor at the rate of 1.16 kg/h (0.53 lb/h). The volume of the reactor was 1.54m³ (55 ft³); the resin's weight inside the reactor was 50.8 kg (112 lbs). The catalyst system employed in this Example was vanadium acetyl acetonate with diethylaluminum chloride as co-catalyst and ethyl trichloroacetate as the promoter. The production rate was 9.08 kg/h (20 lb/h). The product had a Mooney value of 55.

Seventy-five percent of the injected ENB was incorporated into polymers by polymerization. The unreacted remainder of ENB, dissolved into polymers and was equal to 0.66 percent of the polymer's weight. With 50.8 kg (112 lbs) of resins inside the reactor, the total unreacted ENB was 1.63 kg (0.74 lbs). If the unreacted ENB completely evaporated inside the reactor, its partial pressure would be 4.66 kPa (0.6764 psia).

At 40°C the saturation pressure is 15.073 x 10³ kPa (2187.7 psia) for ethylene, 2323 kPa (337.1 psia) for propylene, and 1.80 kPa (0.262 psia), for ENB. Since the partial pressures of ethylene and propylene inside the reactor were much less than their saturation pressures, there was no condensed ethylene or propylene. The calculated partial pressure of unreacted ENB inside the reactor, however, is much higher than its saturation pressure. Therefore, the ENB must have remained in a liquid state and been absorbed by the polymers.

### Examples 3-10

Examples 3-10 set forth in tabular form, operating conditions for producing a variety of different polymers in accordance with the invention. They illustrate the practice of the invention using different catalyst systems and differing cycle gas compositions (Examples 5, 6 and 9 are comparative examples).

| **EXAMPLE NO.** PRODUCT: | 3 POLYBUTADIENE | 4 SBR | 5 ABS | 6 POLYSTYRENE |
|---|---|---|---|---|
| **Reaction Conditions:** | | | | |
| Temperature (°C) | 40 | 40 | 40 | 40 |
| Pressure kPa (psi) | 689(100) | 758(110) | 1378(200) | 689(100) |
| Superficial Velocity m/s (ft/s) | 0.53(1.75) | 0.60(2.0) | 0.46(1.5) | 0.46(1.5) |
| Production Rate kg/h (Ib/h) | 13.6(30) | 11.34(25) | 9.07(20) | 18.1(40) |
| Total Reactor Volume m³ (ft³) | 1.54(55) | 1.54(55) | 1.54(55) | 1.54(55) |
| Reaction Zone Volume m³ (ft³) | 0.21(7.5) | 0.21(7.5) | 0.21(7.5) | 0.21(7.5) |
| Bed Height m (ft) | 2.13(7.0) | 2.13(7.0) | 2.13(7.0) | 2.13(7.0) |
| Bed Diameter m (ft) | 0.36(1.17) | 0.36(1.17) | 0.36(1.17) | 0.36(1.17) |
| Bed Weight kg (lbs) | 50.8(112) | 50.8(112) | 50.8(112) | 50.8(112) |
| **Cycle Gas Composition:** | | | | |
| N2 | 20 | 27.3 | 58.0 | 99.7 |
| Butadiene | 80 | 72.5 | 39.9 | -- |
| Styrene | -- | -2 | 0.15 | 0.3 |
| Acrylonitrile | -- | -- | 1.95 | -- |
| Catalyst | Co(acac)₃* | Co(acac)₃* | Co(acac)₃* | Cp₂ZrMe₂** |
| **Co-catalyst:** | Triethylaluminum | Triethylaluminum | Triethylaluminum | MAO*** |
| Heavy Monomer Feed | | | | |
| Rate kg/h (Ib/h) | | | | |
| Butadiene | 20.9(46.2) | 5.19(9.62) | 0.18(2.46) | -- |
| Styrene | -- | 9.46(20.83) | 6.96(15.33) | 20.16(44.4) |
| Acrylonitrile | -- | | 3.21(7.08) | -- |
| **Polymer Composition:** | | | | |
| Butadiene | 100 | 25 | 8 | -- |
| Styrene | | 75 | 69 | 100 |
| Acrylonitrile | | -- | 23 | -- |

| | | | | |
|---|---|---|---|---|
| * Cobalt triacetylacetonate | | | | |
| ** Dicyclopentadienylzirconiumdimethyl | | | | |
| *** Methylalumoxane | | | | |

### Example 7

In an example of the process of the invention a fluidized bed reaction system as described above, is operated as described below to produce polybutadiene. The polymer is produced under the following reaction conditions: 55°C reactor temperature and 689 kPa (100 psia) total reactor pressure. The partial pressure of the butadiene monomer inside the reactor is 551 kPa (80 psia). The partial pressure of nitrogen is 137.8 kPa (20 psia). The catalyst system employed in this Example is cobalt tris(acetylacetonate). It may be supported on silica or fed as a solution in methylene chloride. Methylaluminoxane is used as cocatalyst. Catalyst and co-catalyst feeds are adjusted to give a 400:1 molar ratio of Al to Co. At steady state the monomer is fed into the reaction system at the rate of 21.7 kg/h (47.8 lb/h). Dried N-650 carbon black is fed to the reactor at the rate of 9.07 kg/h (20 lb/h). Butadiene monomer leaves the reactor at 6.8 Kg/h (15 lb/h) in vent streams. The production rate is 13.6 kg/h (30 lb/h) of polymer after adjusting for the carbon black content. The product has a Mooney viscosity ML (1 + 4 @ 100°C) of 66. Other conditions are shown for Example 7 in the table.

At steady state a total of 21.7 kg/h (47.8 lb/h) butadiene is being fed to the reactor and a total of 20.4 kg/h (45 lb/h) is accounted for leaving the reactor as gas in a vent stream or as polymer. The difference of 1.27 kg/h (2.8 lb/h) must be unreacted liquid butadiene monomer in the polymer leaving the reactor. Since the polymer discharged is identical with the polymer in the bed, the polymer in the bed must contain the same proportion of liquid monomer, i.e. there must be 4.72 kg (10.4 lbs) of dissolved liquid monomer in the 50.8 kg (112 lbs) polymer bed.

The reactor volume is 1.54m² (55 ft³). At the partial pressure of 551 kPa (80 psia), there are 17.07 kg (37.6 lbs) of butadiene in the reactor gas-phase. The total unpolymerized butadiene in the reactor is thus 21.7 kg [48.0 lbs (=37.6 + 10.4)]. If all of this butadiene were in the gas phase of this reactor at once it would have a partial pressure of 716.5 kPa (104 psia) and its condensation temperature would be 61°C. Therefore the reactor at 55°C is being operated below the condensation temperature of the monomer present in the polymerization zone. Furthermore, the presence of this liquid monomer in the gas-phase reactor does not cause agglomeration of the polymer.

| **EXAMPLE NO.** PRODUCT: | 7 POLYBUTADIENE | 8 SBR | 9 ABS | 10 POLYISOPRENE |
|---|---|---|---|---|
| **Reaction Conditions:** | | | | |
| Temperature (°C) | 55 | 55 | 55 | 65 |
| Total Pressure kPa (psia) | 689(100) | 758(110) | 1378(200) | 689(100) |
| Superficial Velocity m/s (ft/s) | 0.53(1.75) | 0.60(2.0) | 0.46(1.5) | 0.53(1.5) |
| Production Rate kg/h (lb/h) | 13.6(30) | 11.34(25) | 9.07(20) | 13.6(30) |
| Total Reactor Volume m³ (ft³) | 1.54(55) | 1.54(55) | 1.54(55) | 1.54(55) |
| Reaction Zone Volume m³ (ft³) | 0.21(7.5) | 0.21(7.5) | 0.21(7.5) | 0.21(7.5) |
| Bed Height m (ft) | 2.13(7.0) | 2.13(7.0) | 2.13(7.0) | 2.13(7.0) |
| Bed Diameter m (ft) | 0.36(1.17) | 0.36(1.17) | 0.36(1.17) | 0.36(1.17) |
| Bed Weight kg (lbs) | 50.8(112) | 50.8(112) | 50.8(112) | 50.8(112) |
| **Cycle Gas Composition:** | | | | |
| N2 | 20 | 27.3 | 58.0 | 70 |
| Butadiene | 80 | 72.5 | 39.9 | -- |
| Styrene | -- | 0.2 | 0.15 | -- |
| Acrylonitrile | -- | -- | 1.95 | -- |
| Isoprene | -- | -- | -- | 30 |
| Catalyst | Co(acac)₃* | CpTiCl₃ | CpTiCl₃ | TiCl₄ |
| **Co-catalyst:** | MAO*** | MAO*** | MAO*** | TEAL** |
| Monomer Feed Rate kg/h (Ib/h) | | | | |
| Butadiene | 21.70(47.8) | 5.19(9.62) | 0.18(2.46) | -- |
| Styrene | -- | 9.46(20.83) | 6.96(15.33) | -- |
| Acrylonitrile | -- | -- | 3.21(7.08) | -- |
| Isoprene | -- | -- | -- | 16.07(35.4) |
| Total Monomer Vent | 6.81(15) | 0.45(1) | 0.45(1) | 0.90(2) |
| Rate kg/h (Ib/h) | | | | |
| **Polymer Composition:** | | | | |
| (wt%) | | | | |
| Butadiene | 100 | 25 | 8 | -- |
| Styrene | -- | 75 | 69 | -- |
| Acrylonitrile | | -- | -- | 100 |

| | | | | |
|---|---|---|---|---|
| * Cobalt triacetylacetonate | | | | |
| ** also Diphenyl Ether | | | | |
| *** Methylalumoxane | | | | |

### Example 11

To a gas-phase stirred bed reactor that was maintained at a constant temperature of 22°C, 1.91 kg (4.2 pounds) of dried carbon black powder were added to act as a fluidization aid. To this were added 0.017 kg (0.039 lbs) ethylaluminum sesquichloride (EASC). Then was added 0.276 kg (0.61 lbs) of 1,3-butadiene and sufficient nitrogen to bring the total reactor pressure to 2170 kPa (315 psia). A small feed of supported CoCl₂(pyridine)₄ catalyst was begun. Simultaneously, a small feed of 10 wt% ethylaluminum sesquichloride co-catalyst solution in isopentane was begun. Feed was adjusted to give a 15:1 molar ratio of Al:Co. During a 2.2 hour polymerization reaction, a total of 3.11 kg (6.84 lbs) of additional butadiene were fed in order to replace butadiene that was polymerized or vented. A small vent stream leaving the reactor removed a total of 0.10 kg (0.22 lbs) butadiene during the polymerization. At the end of the polymerization, the catalyst and co-catalyst feeds were stopped. The reactor was depressurized, and the reactor contents purged free of residual butadiene using nitrogen. The polymer was discharged from the reactor. The product did not contain any lumps that would indicate agglomeration had occurred. On the contrary, the product was a free-flowing, fine, granular powder. The reactor was opened and cleaned to ensure that all product was recovered. The total weight of solid product that was recovered was adjusted for the carbon black that had been initially charged. The remainder 2.60 kg (5.73 lbs) was the amount of butadiene polymer formed during the batch and which was present in the reactor when it was shut down. Since a total of 3.38 kg [7.45 lbs (= 6.84 + 0.61)] of butadiene were charged to the reactor and a total of 2.70 kg [5.95 lbs (= 5.73 + 0.22)] of butadiene have been accounted for leaving the reactor as polymer and in the continuous vent stream, there must have been 0.68 kg (1.50 lbs) of butadiene monomer present in the reactor when polymerization was terminated. This monomer would have been removed from the reactor when it was depressurized and the contents purged.

The reactor volume is 61.7 liters (or 2.18 cubic feet). At 22°C the vapor pressure of 1,3-butadiene is 241.2 kPa(35 psia). The mass of butadiene present in the reactor as a gas at saturation would thus be 0.33 kg (0.73 lbs). Of the total of 0.68 kg (1.50 lbs) of unpolymerized butadiene that was shown to be present in the reactor at shutdown, at most 0.33 kg (0.73 lbs) could have been in the vapor phase and the rest 0.35 kg (0.77 lbs) must have been present in a condensed phase, for example, dissolved in the polymer. Thus the reactor was being operated at a temperature below the condensation temperature of the monomer present. The 0.35 kg (0.77 lbs) of liquid monomer combined with the 2.60 kg (5.73 lbs) of polymer amounts to 6.08 kg (13.4 lbs) of condensed butadiene monomer per 45.4 kg (100 lbs) of polybutadiene. Yet, the presence of this liquid monomer in the gas-phase reactor did not cause agglomeration of the polymer. The table gives further details on this example.

Examples 12-18 were conducted as in Example 11, but with the changes indicated in the table.

Supported Catalyst Preparation for Example 12. To a 500 mL dry nitrogen purged flask is added 31.9 grams of silica (600°C activation) and 7.272 grams of CoCl₂ (pyridine)₄. To this is added 150 mL of CH₂Cl₂. The slurry was stirred for a few minutes and then the solvent was removed under vacuum.

Solution Catalyst Preparation for Example 18. Into a dry nitrogen purged flask is charged 1.648 grams of cobalt tris acetylacetonate. To this is added 100 mL of dry CH₂Cl₂. The mixture is stirred for a few minutes and charged to a pressurizable metal cylinder and fed to the reactor as a solution.

| **EXAMPLE NO.** **PRODUCT** | 11 POLYBUTADIENE | 12 POLYBUTADIENE | 13 POLYBUTADIENE | 14 POLYBUTADIENE |
|---|---|---|---|---|
| **CATALYST DETAILS** | | | | |
| Catalyst | Cobalt dichloridepyridine on silica | Cobalt dichloridepyridine on silica | Cobalt acetyl acetonate on silica | Cobalt dichloridepyridine on silica |
| Co-catalyst | 10% EASC in isopentane | 15% DEACO in toluene | 10% EASC in isopentane | 10% MAO in toluene |
| **PROCESS CONDITIONS** | | | | |
| Reaction Temp. (°C) | 22 | 23 | 20 | 20 |
| BD partial pressure kPa (psia) | 207(30) | 207(30) | 207(30) | 207(30) |
| Polymer produced kg (lb) | 2.59(5.7) | 2.86(6.3) | 2.45(5.4) | 2.63(5.8) |
| Reaction time | 2 hr 10 min | 3 hr | 2 hr 15 min | 1 hr 20 min |
| **PRODUCT ANALYSIS** | | | | |
| % Carbon Black N-650 analysis | 44 | 38 | 44 | 45 |
| Average particle size by sieve analysis cm (inch) | 0.049(0.016) | 0.048(0.019) | 0.038(0.015) | 0.086(0.034) |
| Aluminum/ Catalyst feed ratio* | 15 | 28 | 11 | 607 |
| Cobaltcontent in the polymer (ppm) | 55 | 81 | 94 | 19 |
| Reduced Viscosity (dl/g) | 1.5 | 1.0 | 1.0 | 3.6 |
| Mooney viscosity ML (1 + 4 @ 100°C) | | 42 | | |
| % cis -1,4 | 93 | 92 | 92 | 98.4 |

| | | | | |
|---|---|---|---|---|
| * molar ratio of Al to transition metal in continuous feeds | | | | |

| **EXAMPLE NO.** **PRODUCT** | 15 POLYBUTADIENE | 16 POLYBUTADIENE | 17 POLYBUTADIENE | 18 POLYBUTADIENE |
|---|---|---|---|---|
| **CATALYST DETAILS** | | | | |
| Catalyst | Cobalt dichloride pyridine on silica | Cobalt dichloride pyridine- IPPD diamine on silica | Cobalt octoate on silica | Cobalt acetyl acetonate in methylene chloride |
| | | | | |
| Co-catalyst | 10% MAO in toluene | 15% EASC in toluene | 15% DEACO in toluene | 10% DEAC in isopentane |
| PROCESS CONDITIONS | | | | |
| Reaction | 20 | 20 | 20 | 20 |
| Temp. (°C) partial pressure kPa (psia) | 207(30) | 207(30) | 207(30) | 172(25) |
| Polymer produced kg (lb) | 1.91(4.2) | 2.95(6.5) | 3.09(6.8) | 2.59(5.7) |
| Reaction time | 1 hr | 4 hr 30 min | 3 hr 10 min | 4 hr 30 min |
| **PRODUCT ANALYSIS** | | | | |
| % Carbon Black N-650 analysis | 56 | 44 | 41 | 44 |
| Average particle size by sieve analysis cm inch) | 0.091(0.036) | 0.040(0.016) | 0.033(0.013) | Size not measured |
| AVCatalyst feed ratio* | 385 | 62 | 10 | 45 |
| Cobaltcontent in the polymer (ppm) | 45 | 84 | 195 | 45 |
| Reduced Viscosity (dVg) | 1.0 | 1.1 | 1.0 | 0.7 |
| Mooney viscosity ML (1 + 4 @ 100°C) | 40 | | | |
| % cis-1,4 | 95.7 | 96 | 92.1 | 90 |

| | | | | |
|---|---|---|---|---|
| t N-isopropyl-N'-phenyl-p-phenylenediamine was present on the catalyst at 15 moles per mole of cobalt. * molar ratio of Al to transition metal in continuous feeds | | | | |

| **EXAMPLE NO.** **PRODUCT** | 19 POLYBUTADIENE | 20 POLYBUTADIENE | 21 POLYISOPRENE |
|---|---|---|---|
| **CATALYST DETAILS** | | | |
| Catalyst | Cyclopentadiene titanium trichloride | Nickel octoate | TiCl₄ diphenyl-ether |
| | | | |
| Co-catalyst | 10% MAO in toluene | 10% TEAL 10% BF₃ etherate | TIBA |

| **PROCESS CONDITIONS** | | | |
|---|---|---|---|
| Reaction Temperature (°C) | 50 | 50 | 50 |
| Monomer partial pressure kPa (psia) | 413(60) | 413(60) | 172(25) |
| Reaction time | 2 hr | 4 hr | 4 hr |

| **PRODUCT ANALYSIS** | | | |
|---|---|---|---|
| % Carbon Black N-650 by analysis | 40 | 40 | 40 |
| Co-catalyst/ Catalyst feed ratio* | 500 | 60 | 10 |

| | | | |
|---|---|---|---|
| * molar ratio of Al to transition metal in continuous feeds | | | |

## Claims

1. A process for producing polybutadiene or polyisoprene in a stirred bed or gas fluidized polymerization vessel having a polymerization zone under polymerization reaction conditions, which process comprises:
(i) introducing butadiene or isoprene monomer into said polymerization zone containing a bed of growing polymer particles in the presence of an inert particulate material and optionally at least one inert gas;
(ii) continuously or intermittently introducing a polymerization catalyst containing a metal component of nickel, cobalt, titanium, or mixtures thereof, a co-catalyst, and optionally a promoter into said polymerization zone;
(iii) continuously or intermittently withdrawing polybutadiene or polyisoprene product from said polymerization zone; and
(iv) withdrawing unreacted butadiene or isoprene from said polymerization zone, compressing and cooling said butadiene or isoprene and said inert gas when present, while maintaining the temperature within said polymerization zone below the dew point of the monomer present in said polymerization zone.

2. A process as claimed in Claim 1 wherein the temperature within the polymerization zone is maintained below the condensation temperature of the monomer present in the polymerization zone.

3. A process as claimed in Claim 1 wherein the conditions within said polymerization zone are such that essentially no liquid is present in the polymerization zone that is not adsorbed on or adsorbed in solid particulate matter.

4. A process as claimed in Claim 1 wherein the conditions within the polymerization zone are such that at least a portion of the monomer is a liquid that is not absorbed in the solid particulate matter.

5. A process as claimed in any one of the preceding claims wherein the polymerization process is conducted in the presence of an inert particulate material which is carbon black, silica, clay, talc or a mixture thereof.

6. A process as claimed in any one of the preceding claims wherein the polymerization catalyst contains a nickel metal component; the co-catalyst is triethylaluminum, triisobutylaluminum, diethyl aluminum chloride, partially hydrolyzed diethylaluminum chloride, methylaluminoxane, modified methylaluminoxane, or a mixture thereof; and the promoter is hydrogen fluoride, boron trifluoride, an etherate of hydrogen fluoride, an etherate of boron trifluoride, or a mixture thereof.

7. A process as claimed in any one of Claims 1 to 5 wherein the polymerization catalyst contains a titanium metal component; the co-catalyst is triethylaluminum, triisobutylaluminium, dialkylaluminum iodide, methylaluminoxane, or a mixture thereof; and optionally the promoter is iodine, an organic etherate, or a mixture thereof.

8. A process as claimed in any one of Claims 1 to 5 wherein the polymerization catalyst contains a cobalt metal component; and the co-catalyst is ethylaluminum sesquichloride, ethyl aluminum dichloride, partially hydrolyzed diethylaluminum chloride, diisobutyl aluminum chloride, partially hydrolyzed diisobutyl aluminum chloride, diethylaluminum chloride, methylaluminoxane, or a mixture thereof.

9. A process as claimed in claim 8 wherein the polymerization catalyst additionally comprises water as a promoter.

10. A process as claimed in any one of the preceding claims wherein the polymerization catalyst is supported on silica, carbon black, porous crosslinked polystyrene, porous crosslinked polypropylene, alumina, thoria, zirconia, magnesium chloride, or a mixture thereof.

11. A process as claimed in any one of the preceding claims wherein butadiene or isoprene are introduced continuously into the polymerization zone.

12. A process as claimed in any one of the preceding claims wherein the process is conducted in the presence of an agent or device for controlling the level of static in said reactor.

13. A process as claimed in Claim 12 wherein the static voltage in said reactor is maintained essentially neutral.

14. A process as claimed in any one of the preceding claims wherein the inert gas is nitrogen.

15. A process as claimed in any one of the preceding claims wherein the polymerization catalyst is spray dried.

16. A process as claimed in any one of Claims 1 to 14 wherein the polymerization catalyst is introduced to the polymerization zone as a prepolymer.

17. A process as claimed in any one of Claims 1 to 14 wherein the polymerization catalyst introduced to the polymerization zone is a liquid, a solution, or a slurry.

18. A process for producing styrene-butadiene rubber in a stirred bed or gas fluidized polymerization vessel having a polymerization zone under polymerization reaction conditions, which process comprises:
(1) introducing styrene and butadiene monomers into said polymerization zone containing a bed of growing polymer particles in the presence of an inert particulate material and optionally at least one inert gas;
(ii) continuously or intermittently introducing a metallocene polymerization catalyst, a co-catalyst, and optionally a promoter into said polymerization zone;
(iii) continuously or intermittently withdrawing styrene-butadiene rubber product from said polymerization zone; and
(iv) withdrawing unreacted butadiene and styrene from said polymerization zone, compressing and cooling said butadiene and styrene and said inert gas when present, while maintaining the temperature within said polymerization zone below the dew point of at least one of the monomers present in said polymerization zone.

19. A process as claimed in Claim 18 wherein the temperature within the polymerization zone is maintained below the condensation temperature of at least one of the monomers present in the polymerization zone.

20. A process as claimed in Claim 18, wherein the conditions within the polymerization zone are such that essentially no liquid is present in said polymerization zone that is not adsorbed on or absorbed in solid particulate matter.

21. A process as claimed in Claim 18, wherein the conditions within the polymerization zone are such that at least a portion of the monomers is a liquid that is not absorbed in the solid particulate matter.

22. A process as claimed in any one of Claims 18 to 21, wherein the polymerization process is conducted in the presence of inert particulate material which is carbon black, silica, clay, talc or a mixture thereof.

23. A process as claimed in Claim 21 or Claim 22 wherein the co-catalyst is methylaluminoxane.

## Patentansprüche

1. Verfahren zur Herstellung von Polybutadien oder Polyisopren in einem Rührbett- oder Gas-Wirbelschicht-Polymerisationsreaktor mit einer Polymerisationszone unter Polymerisationsreaktionsbedingungen, worin das Verfahren umfaßt:
(i) Einleiten von Butadien- oder Isopren-Monomer in die Polymerisationszone, die ein Bett von wachsenden Polymerteilchen enthält, in Anwesenheit eines inerten teilchenförmigen Materials und gegebenenfalls mindestens eines Inertgases;
(ii) kontinuierliches oder diskontinuierliches Einführen eines Polymerisationskatalysators, der eine Metallkomponente von Nickel, Kobalt, Titan oder Mischungen derselben, einen Cokatalysator und gegebenenfalls einen Promotor enthält, in die Polymerisationszone;
(iii) kontinuierliche oder diskontinuierliche Entnahme von Polybutadien- oder Polyisopren-Produkt aus der Polymerisationszone; und
(iv) Abziehen von nicht umgesetztem Butadien oder Isopren aus der Polymerisationszone, Komprimieren und Abkühlen des Butadiens oder Isoprens und, falls vorhanden, des Inertgases, während die Temperatur innerhalb der Polymerisationszone unterhalb des Taupunkts des in der Polymerisationszone vorhandenen Monomers gehalten wird.

2. Verfahren wie in Anspruch 1 beansprucht, worin die Temperatur innerhalb der Polymerisationszone unterhalb der Kondensationstemperatur des in der Polymerisationszone vorhandenen Monomers gehalten wird.

3. Verfahren wie in Anspruch 1 beansprucht, worin die Bedingungen innerhalb der Polymerisationszone derart sind, daß in der Polymerisationszone im wesentlichen keine Flüssigkeit vorhanden ist, die nicht auf festem Teilchenmaterial adsorbiert oder darin absorbiert ist.

4. Verfahren wie in Anspruch 1 beansprucht, worin die Bedingungen innerhalb der Polymerisationszone so sind, daß mindestens ein Teil des Monomers eine Flüssigkeit ist, die nicht in dem festen Teilchenmaterial absorbiert ist.

5. Verfahren wie in irgendeinem der vorhergehenden Ansprüche beansprucht, worin das Polymerisationsverfahren in Anwesenheit eines inerten teilchenförmigen Materials ausgeführt wird, das Ruß, Siliciumdioxid, Ton, Talk oder eine Mischung davon ist.

6. Verfahren wie in irgendeinem der vorhergehenden Ansprüche beansprucht, worin der Polymerisationskatalysator eine Nickel-Metallkomponente enthält; der Cokatalysator Triethylaluminium, Triisobutylaluminium, Diethylaluminiumchlorid, teilweise hydrolysiertes Diethylaluminiumchlorid, Methylalumoxan, modifiziertes Methylalumoxan oder eine Mischung davon ist; und der Promotor Fluorwasserstoff, Bortrifluorid, ein Fluorwasserstoff-Etherat, ein Bortrifluorid-Etherat oder eine Mischung davon ist.

7. Verfahren wie in irgendeinem der Ansprüche 1 bis 5 beansprucht, worin der Polymerisationskatalysator eine Titan-Metallkomponente enthält; der Cokatalysator Triethylaluminium, Triisobutylaluminium, Dialkylaluminiumiodid, Methylalumoxan oder eine Mischung davon ist; und gegebenenfalls der Promotor lod, ein organisches Etherat oder eine Mischung davon ist.

8. Verfahren wie in irgendeinem der Ansprüche 1 bis 5 beansprucht, worin der Polymerisationskatalysator eine Kobalt-Metallkomponente enthält; und der Cokatalysator Ethylaluminiumsesquichlorid, Ethylaluminiumdichlorid, teilweise hydrolysiertes Diethylaluminiumchlorid, Diisobutylaluminiumchlorid, teilweise hydrolysiertes Diisobutylaluminiumchlorid, Diethylaluminiumchlorid, Methylalumoxan oder eine Mischung davon ist.

9. Verfahren wie in Anspruch 8 beansprucht, worin der Polymerisationskatalysator zusätzlich Wasser als Promotor umfaßt.

10. Verfahren wie in irgendeinem der vorhergehenden Ansprüche beansprucht, worin der Polymerisationskatalysator auf Siliciumdioxid, Ruß, porösem vernetztem Polystyrol, porösem vernetztem Polypropylen, Aluminiumoxid, Thoriumoxid, Zirkonoxid, Magnesiumchlorid oder einer Mischung davon geträgert ist.

11. Verfahren wie in irgendeinem der vorhergehenden Ansprüche beansprucht, worin Butadien oder Isopren kontinuierlich in die Polymerisationszone eingeleitet werden.

12. Verfahren wie in irgendeinem der vorhergehenden Ansprüche beansprucht, worin das Verfahren in Anwesenheit eines Mittels oder einer Vorrichtung zur Steuerung der elektrostatischen Ladung im Reaktor ausgeführt wird.

13. Verfahren wie in Anspruch 12 beansprucht, worin die elektrostatische Spannung im Reaktor im wesentlichen neutral gehalten wird.

14. Verfahren wie in irgendeinem der vorhergehenden Ansprüche beansprucht, worin das Inertgas Stickstoff ist.

15. Verfahren wie in irgendeinem der vorhergehenden Ansprüche beansprucht, worin der Polymerisationskatalysator sprühgetrocknet ist.

16. Verfahren wie in irgendeinem der Ansprüche 1 bis 14 beansprucht, worin der Polymerisationskatalysator als Prepolymer in die Polymerisationszone eingeführt wird.

17. Verfahren wie in irgendeinem der Ansprüche 1 bis 14 beansprucht, worin der in die Polymerisationszone eingeführte Polymerisationskatalysator eine Flüssigkeit, eine Lösung oder eine Aufschlämmung ist.

18. Verfahren zur Herstellung von Styrol-Butadien-Kautschuk in einem Rührbett- oder Gas-Wirbelschicht-Polymerisationsreaktor mit einer Polymerisationszone unter Polymerisationsreaktionsbedingungen, worin das Verfahren umfaßt:
(i) Einleiten von Styrol- und Butadien-Monomeren in die Polymerisationszone, die ein Bett wachsender Polymerteilchen enthält, in Anwesenheit eines inerten teilchenförmigen Materials und gegebenenfalls mindestens eines Inertgases;
(ii) kontinuierliches oder diskontinuierliches Einführen eines Metallocen-Polymerisationskatalysators, eines Cokatalysators und gegebenenfalls eines Promotors in die Polymerisationszone;
(iii) kontinuierliche oder diskontinuierliche Entnahme von Styrol-Butadien-Kautschuk-Produkt aus der Polymerisationszone; und
(iv) Abziehen von nicht umgesetztem Butadien und Styrol aus der Polymerisationszone, Komprimieren und Abkühlen des Butadiens und Styrols und, falls vorhanden, des Inertgases, während die Temperatur innerhalb der Polymerisationszone unterhalb des Taupunkts mindestens eines der in der Polymerisationszone vorhandenen Monomere gehalten wird.

19. Verfahren wie in Anspruch 18 beansprucht, worin die Temperatur innerhalb der Polymerisationszone unterhalb der Kondensationstemperatur mindestens eines der in der Polymerisationszone vorhandenen Monomere gehalten wird.

20. Verfahren wie in Anspruch 18 beansprucht, worin die Bedingungen innerhalb der Polymerisationszone so sind, daß in der Polymerisationszone im wesentlichen keine Flüssigkeit vorhanden ist, die nicht auf festem Teilchenmaterial adsorbiert oder in festem Teilchenmaterial absorbiert ist.

21. Verfahren wie in Anspruch 18 beansprucht, worin die Bedingungen innerhalb der Polymerisationszone so sind, daß mindestens ein Teil der Monomere eine Flüssigkeit ist, die nicht in dem festen Teilchenmaterial absorbiert ist.

22. Verfahren wie in irgendeinem der Ansprüche 18 bis 21 beansprucht, worin das Polymerisationsverfahren in Anwesenheit eines inerten teilchenförmigen Materials ausgeführt wird, das Ruß, Siliciumdioxid, Ton, Talk oder eine Mischung davon ist.

23. Verfahren wie in Anspruch 21 oder Anspruch 22 beansprucht, worin der Cokatalysator Methylalumoxan ist.

## Revendications

1. Procédé pour la production de polybutadiène ou de polyisoprène dans un récipient de polymérisation à lit sous agitation ou à fluidisation en phase gazeuse, comprenant une zone de polymérisation, dans des conditions de réaction de polymérisation, procédé qui comprend les étapes consistant :
(i) à introduire un monomère butadiène ou isoprène dans ladite zone de polymérisation contenant un lit de particules de polymère en croissance, en présence d'une matière inerte en particules et, facultativement, d'au moins un gaz inerte ;
(ii) à introduire de manière continue ou intermittente un catalyseur de polymérisation contenant un métal consistant en nickel, cobalt, titane ou un de leurs mélanges, un cocatalyseur et, facultativement, un promoteur dans ladite zone de polymérisation ;
(iii) à décharger de manière continue ou intermittente le produit consistant en polybutadiène ou polyisoprène de ladite zone de polymérisation ; et
(iv) à décharger le butadiène ou l'isoprène n'ayant pas réagi de ladite zone de polymérisation, à comprimer et refroidir ledit butadiène ou isoprène et ledit gaz inerte lorsqu'il est présent, tout en maintenant la température dans ladite zone de polymérisation au-dessous du point de rosée du monomère présent dans ladite zone de polymérisation.

2. Procédé suivant la revendication 1, dans lequel la température dans la zone de polymérisation est maintenue au-dessous de la température de condensation du monomère présent dans la zone de polymérisation.

3. Procédé suivant la revendication 1, dans lequel les conditions dans la zone de polymérisation sont telles qu'il n'existe dans cette zone de polymérisation pratiquement aucune quantité de liquide qui ne soit adsorbée sur, ou absorbée dans, la matière solide en particules.

4. Procédé suivant la revendication 1, dans lequel les conditions dans la zone de polymérisation sont telles qu'au moins une partie du monomère consiste en un liquide qui n'est pas absorbé dans la matière solide en particules.

5. Procédé suivant l'une quelconque des revendications précédentes, dans lequel le procédé de polymérisation est mis en oeuvre en présence d'une matière inerte en particules, qui consiste en noir de carbone, silice, argile, talc ou un de leurs mélanges.

6. Procédé suivant l'une quelconque des revendications précédentes, dans lequel le catalyseur de polymérisation contient un métal consistant en nickel ; le cocatalyseur consiste en triéthylaluminium, triisobutylaluminium, chlorure de diéthylaluminium, chlorure de diéthylaluminium partiellement hydrolysé, méthylaluminoxane, méthylaluminoxane modifié ou un de leurs mélanges ; et le promoteur consiste en fluorure d'hydrogène, trifluorure de bore, un complexe d'éther de fluorure d'hydrogène, un complexe d'éther de trifluorure de bore ou un de leurs mélanges.

7. Procédé suivant l'une quelconque des revendications 1 à 5, dans lequel le catalyseur de polymérisation contient un métal consistant en titane ; le cocatalyseur consiste en triéthylaluminium, triisobutylaluminium, un iodure de dialkylaluminium, méthylaluminoxane ou un de leurs mélanges; et, facultativement, le promoteur consiste en iode, un complexe d'éther organique ou un de leurs mélanges.

8. Procédé suivant l'une quelconque des revendications 1 à 5, dans lequel le catalyseur de polymérisation contient un métal consistant en cobalt ; et le cocatalyseur consiste en sesquichlorure d'éthylaluminium, dichlorure d'éthylaluminium, chlorure de diéthylaluminium partiellement hydrolysé, chlorure de diisobutylaluminium, chlorure de diisobutylaluminium partiellement hydrolysé, chlorure de diéthylaluminium, méthylaluminoxane ou un de leurs mélanges.

9. Procédé suivant la revendication 8, dans lequel le catalyseur de polymérisation comprend en outre de l'eau comme promoteur.

10. Procédé suivant l'une quelconque des revendications précédentes, dans lequel le catalyseur de polymérisation est fixé sur un support consistant en silice, noir de carbone, polystyrène réticulé poreux, polypropylène réticulé poreux, alumine, oxyde de thorium, zircone, chlorure de magnésium ou un de leurs mélanges.

11. Procédé suivant l'une quelconque des revendications précédentes, dans lequel du butadiène ou de l'isoprène est introduit de manière continue dans la zone de polymérisation.

12. Procédé suivant l'une quelconque des revendications précédentes, qui est mis en oeuvre en présence d'un agent ou dispositif pour ajuster la quantité de charge statique dans le réacteur.

13. Procédé suivant la revendication 12, dans lequel la tension statique dans le réacteur est maintenue pratiquement à la neutralité.

14. Procédé suivant l'une quelconque des revendications précédentes, dans lequel le gaz inerte est l'azote.

15. Procédé suivant l'une quelconque des revendications précédentes, dans lequel le catalyseur de polymérisation est séché par pulvérisation.

16. Procédé suivant l'une quelconque des revendications 1 à 14, dans lequel le catalyseur de polymérisation est introduit sous forme d'un prépolymère dans la zone de polymérisation.

17. Procédé suivant l'une quelconque des revendications 1 à 14, dans lequel le catalyseur de polymérisation introduit dans la zone de polymérisation est un liquide, une solution ou une suspension.

18. Procédé pour la production d'un caoutchouc styrène-butadiène dans un récipient de polymérisation à lit sous agitation eu à fluidisation en phase gazeuse, comprenant une zone de polymérisation, dans des conditions de polymérisation, procédé qui comprend les étapes consistant :
(i) à introduire des monomères styrène et butadiène dans ladite zone de polymérisation contenant un lit de particules de polymère en croissance, en présence d'une matière inerte en particules et, facultativement, d'au moins un gaz inerte ;
(ii) à introduire de manière continue ou intermittente un catalyseur de polymérisation à base d'un métallocène, un cocatalyseur et, facultativement, un promoteur dans ladite zone de polymérisation ;
(iii) à décharger de manière continue ou intermittente le produit consistant en caoutchouc styrène-butadiène de ladite zone de polymérisation ; et
(iv) à décharger le butadiène et le styrène n'ayant pas réagi de ladite zone de polymérisation, à comprimer et refroidir ledit butadiène et ledit styrène ainsi que ledit gaz inerte lorsqu'il est présent ; tout en maintenant la température dans ladite zone de polymérisation au-dessous du point de rosée d'au moins un des monomères présents dans ladite zone de polymérisation.

19. Procédé suivant la revendication 18, dans lequel la température dans la zone de polymérisation est maintenue au-dessous de la température de condensation d'au moins un des monomères présents dans la zone de polymérisation.

20. Procédé suivant la revendication 18, dans lequel les conditions dans la zone de polymérisation sont telles qu'il n'existe dans ladite zone de polymérisation pratiquement aucune quantité de liquide qui ne soit pas adsorbée sur ou absorbée dans la matière solide en particules.

21. Procédé suivant la revendication 18, dans lequel les conditions dans la zone de polymérisation sent telles qu'au moins une partie des monomères consiste en un liquide qui n'est pas absorbé dans la manière solide en particules.

22. Procédé suivant l'une quelconque des revendications 18 à 21, dans lequel le procédé de polymérisation est mis en oeuvre en présence d'une matière inerte en particules qui consiste en noir de carbone, silice, argile, talc ou un de leurs mélanges.

23. Procédé suivant la revendication 21 ou la revendication 22, dans lequel le cocatalyseur consiste en méthylaluminoxane.
